# EUROPEAN PATENT APPLICATION

(11) **EP 4 667 944 A1**
(43) Date of publication of application: **24.12.2025**
(21) Application number: 24755840.6
(22) Date of filing: 08.01.2024
(51) Int. Cl.: G01N 35/04, G01N 35/00, G01N 35/10, G01N 33/53

(54) **SAMPLE RACK MANIPULATION DEVICE, SAMPLE ANALYZER, AND METHOD FOR SAMPLE COLLECTION**

(30) Priority: 14.02.2023 CN 202310111277
(71) Applicant: Beckman Coulter Laboratory Systems (Suzhou) Co. Ltd., Suzhou, Jiangsu 215126 (CN)
(72) Inventor: ZHAO, Liang, Suzhou, Jiangsu 215126 (CN)
(74) Representative: Maiwald GmbH
(86) International application number: PCT/CN2024/071055
(87) International publication number: WO 2024/169464

(57) **Abstract**

The present application relates to a sample rack manipulation device for a sample analyzer, a sample analyzer comprising the sample rack manipulation device, and a method for sample collection in a sample analyzer by the sample rack manipulation device. The sample rack manipulation device comprises: a first engagement portion, which can be engaged with a first sample rack; a second engagement portion, which can be engaged with a second sample rack; and a driven portion, which can be engaged with a driving device and is driven by the driving device. The sample rack manipulation device in the present application has a small size, a compact structure, and low costs, and can improve the sample rack transfer efficiency to meet throughput requirements of the sample analyzer.

## Description

### TECHNICAL FIELD

The present application relates to the medical technical field. Specifically, the present application relates to a channel bridging device for sample collection in a sample analyzer, a sample analyzer including the channel bridging device, and a method for sample collection in the sample analyzer through the channel bridging device.

### BACKGROUND

The contents of this section provide only background information relevant to the present disclosure, which may not constitute the prior art.

Various analytical detection instruments (also referred as "sample analyzers" herein) for detecting samples in sample containers such as test tubes or cups are indispensable in the medical field, especially clinical medicine and laboratory medicine. The sample analyzer includes a sample loading/unloading unit, a sample processing unit, and a sampling unit positioned between the sample loading/unloading unit and the sample processing unit.

The sampling unit includes a sampling area and a sampling device with a sampling probe. During sampling, a shuttle loads a sample rack into the sampling area and unloads it to the sample loading/unloading unit after the sampling is completed for this sample rack. Subsequently, the shuttle loads the next sample rack into the sampling area and unloads it to the sample loading/unloading unit after the sampling is completed for this sample rack. This process is repeated for the sample racks to be detected. Thus, it can be seen that the sample racks may only be transferred one by one in the sampling area, meaning that loading, sampling, and unloading operations for one sample rack must be completed before loading, sampling, and unloading operations for another sample rack.

### SUMMARY

In conventional sample analyzers, since sample racks can only be processed one by one in the sampling area, the processing efficiency of the sample racks is limited, and consequently, the detection throughput of the sample analyzer is also restricted.

In view of the aforementioned issues with the conventional sample analyzers, the inventors of the present application proposed an improved solution capable of processing two sample racks simultaneously. Specifically, a sample rack manipulation device is proposed that can simultaneously load one sample rack and unload another sample rack in the sampling area. Such sample rack manipulation device can obviously enhance the transfer efficiency of the sample racks, thereby significantly increasing the detection throughput of the sample analyzer.

According to an aspect of the present disclosure, a sample rack manipulation device for a sample analyzer is provided. The sample rack manipulation device includes: a first engagement portion which can be engaged with a first sample rack; a second engagement portion which can be engaged with a second sample rack; and a driven portion which can be engaged with a driving device and driven by the driving device.

Through this sample rack manipulation device, the first sample rack and the second sample rack can be simultaneously manipulated via the first engagement portion and second engagement portion. Compared to the conventional technology, which can only manipulate one sample rack, it is evident that the sample rack manipulation device according to the present application can significantly improve the operational efficiency of the sample racks, sampling efficiency, and the throughput of the sample analyzer.

In some embodiments, the first engagement portion is adapted to be engaged with a distal end of the first sample rack, and the second engagement portion is adapted to be engaged with a proximal end of the second sample rack. In this way, the sample rack manipulation device can be engaged with the front end or rear end of the adjacent sample racks, thereby occupying a smaller space.

In some embodiments, the first engagement portion and the second engagement portion are arranged in a back-to-back configuration. In this way, the sample rack manipulation device can be more compact in structure.

In some embodiments, the first engagement portion and the second engagement portion are hook-shaped members that are engaged with the first sample rack and the second sample rack, respectively.

In some embodiments, the sample rack manipulation device further includes a sensor for sensing position. This can enhance the operational accuracy and safety of the sample rack manipulation device.

According to another aspect of the present application, a sample analyzer is provided, including the aforementioned sample rack manipulation device.

In some embodiments, the sample analyzer further includes a driving device for driving the sample rack manipulation device. The driving device includes a stepping motor that drives the sample rack manipulation device via a timing belt. With the stepping motor, bidirectional operation of the sample racks can be achieved, making the operation more flexible and convenient.

In some embodiments, the sample rack manipulation device is configured to operate sample racks on an aspiration channel of the sample analyzer. The aspiration channel is linear and includes an aspiration area, a loading/unloading area, and a buffer area sequentially.

According to yet another aspect of the present disclosure, a method for sample collection in a sample analyzer through the aforementioned sample rack manipulation device is provided, including the following steps: moving a first sample rack to an aspiration area of an aspiration channel of the sample analyzer by the sample rack manipulation device to collect samples, where a first engagement portion of the sample rack manipulation device is engaged with the first sample rack; after the sample collection from the first sample rack is completed, moving the first sample rack to a buffer area of the aspiration channel; loading a second sample rack into a loading/unloading area of the aspiration channel and engaging a second engagement portion of the sample rack manipulation device with the second sample rack; moving the first sample rack and the second sample rack by the sample rack manipulation device such that the first sample rack reaches the loading/unloading area; unloading the first sample rack from the loading/unloading area; and moving the second sample rack to the aspiration area by the sample rack manipulation device to collect samples.

In some embodiments, the method further includes: positioning the first sample rack or the second sample rack by the sample rack manipulation device. In this way, a dedicated device for positioning sample racks can be eliminated, and thus costs can be reduced.

In some embodiments, the positioning is achieved by abutting the first engagement portion or the second engagement portion of the sample rack manipulation device against the first sample rack or the second sample rack.

In some embodiments, the step of the positioning includes the followings: moving the first sample rack or second sample rack a first predetermined distance in a first direction by the sample rack manipulation device; and moving the first sample rack or the second sample rack a second predetermined distance in a second direction opposite to the first direction by the sample rack manipulation device, wherein the first predetermined distance and the second predetermined distance are greater than a gap between the first engagement portion or the second engagement portion of the sample rack manipulation device and the first sample rack or the second sample rack.

In some embodiments, the first predetermined distance is equal to the second predetermined distance.

In some embodiments, the first engagement portion and the second engagement portion are engaged with a distal end of the first sample rack and a proximal end of the second sample rack, respectively, which are adjacent to each other.

Other application fields will become apparent from the description provided herein. It should be understood that the specific examples and embodiments described in this section are for illustration purposes only and are not intended to limit the scope of the present disclosure.

### BRIEF DESCRIPTION OF THE DRAWINGS

Features and advantages of one or several embodiments of the present disclosure will become more easily understood from the following description with reference to the accompanying drawings, in which:
FIG. 1 is a perspective schematic view of a sample analyzer according to an embodiment of the present disclosure, with a cover opened for observing an internal structure of the sample analyzer;
FIG. 2 is a partially enlarged schematic view of the sample analyzer in FIG. 1, viewed from a right side;
FIG. 3 is a schematic view illustrating an aspiration channel of the sample analyzer;
FIGS. 4a to 4f illustrate a process of a sample rack manipulation device according to an embodiment of the present disclosure operating on two sample racks during sampling;
FIG. 5 is a perspective schematic view of a sample rack manipulation device according to a first embodiment of the present disclosure;
FIG. 6 is a schematic view illustrating an engagement of the sample rack manipulation device in FIG. 5 with two sample racks;
FIGS. 7a to 7c are schematic views illustrating positioning of the sample racks by the sample rack manipulation device; and
FIG. 8 is a schematic view of a portion of a sample analyzer provided with the sample rack manipulation device.

### DETAILED DESCRIPTION OF THE EMBODIMENTS

Exemplary embodiments will now be described more fully with reference to the accompanying drawings. Corresponding reference numerals in the accompanying drawings indicate the same or corresponding components or features throughout.

Exemplary embodiments are provided so that the present disclosure will be exhaustive and will more fully convey the scope to those skilled in the art. Many specific details such as examples of specific components, devices, and methods are set forth to provide a thorough understanding of various embodiments of the present disclosure. It will be apparent to those skilled in the art that the exemplary embodiments may be implemented in many different forms without specific details, and should not be construed as limiting the scope of the present disclosure. In some exemplary embodiments, well-known processes, well-known device structures, and well-known technologies are not described in detail.

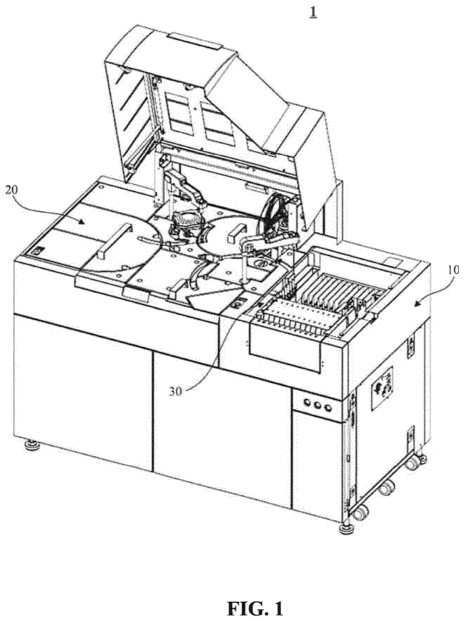

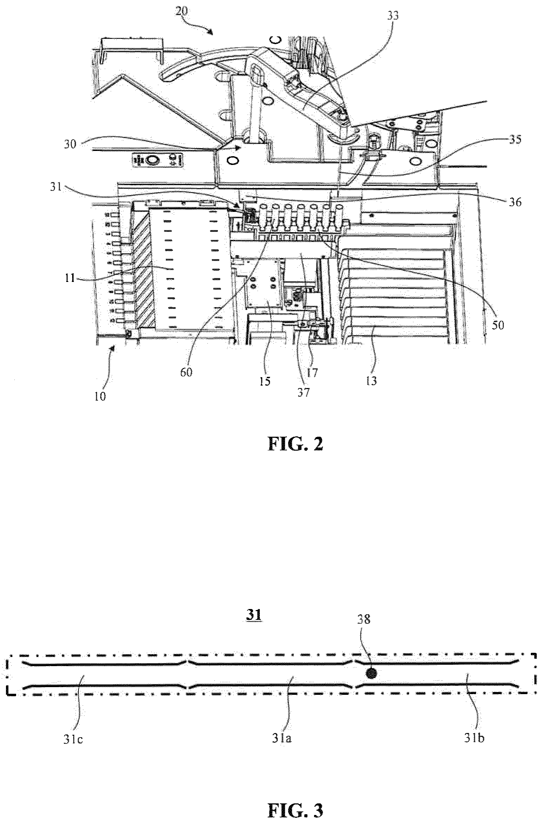

A sample analyzer 1 according to an embodiment of the present disclosure will be described below with reference to FIGS. 1 and 2. It should be understood that the sample analyzer 1 shown in the figures is for illustrative purposes only and should not be limited to the specific examples shown in the figures. The sample analyzer according to the present disclosure may be any suitable sample analyzer for testing or processing biological samples or other chemical samples, for example, a clinical chemical detector or an immunoassay detector.

FIG. 1 is a perspective schematic view of a sample analyzer 1 according to an embodiment of the present disclosure, with a cover opened for observing an internal structure of the sample analyzer, and FIG. 2 is a partially enlarged schematic view of the sample analyzer 1 in FIG. 1, viewed from a right side. As shown in FIGS. 1 and 2, the sample analyzer 1 includes a sample loading/unloading unit 10, a sample processing unit 20, and a sampling unit 30. The sample loading/unloading unit 10 is configured to load or unload a sample rack 50, on which sample containers 60 containing samples are carried. The sample processing unit 20 is configured to process or detect the samples. The sampling unit 30 is configured to aspirate samples from the sample containers 60 and transfer the samples to the sample processing unit 20 for processing or detection.

The aforementioned units of the sample analyzer 1 can be independent of each other or integrated into one piece. The structures of the aforementioned units of the sample analyzer 1 can vary depending on the type of the sample analyzer. For example, sample processing units 20 of the same type or different types, as well as sampling units 30 of the same type or different types, may be arranged on both sides of the sample loading/unloading unit 10 respectively.

The sample loading/unloading unit 10 includes a loading/unloading zone 11, a buffer zone 13, and a transfer zone 15. Operators can load the sample rack 50 into the loading/unloading zone 11 or remove the sample rack 50 from the loading/unloading zone 11. A shuttle 17 moves in the transfer zone 15 to transport the sample rack 50 among the loading/unloading zone 11, the buffer zone 13, and the sampling unit 30. The buffer zone 13 is a region for temporarily placing the sample rack 50 to await the next execution process (e.g., detection, unloading, or obtaining detection results).

The sampling unit 30 includes an aspiration channel 31, a sampling device 33, a sample rack manipulation device 36, and a channel bridging device 37. The aspiration channel 31 is a spatial region where the sample rack 50 is located during sampling. The sampling device 33 has a sampling probe 35. The sampling probe 35 can aspirate samples from the sample containers 60, store the samples, and release the samples when reaching a predetermined position in the sample processing unit 20. The channel bridging device 37 is configured to connect various regions of the aspiration channel 31 to move the sample rack 50. The sample rack manipulation device 36 can operate on two sample racks simultaneously to efficiently transfer the sample racks (as described in detail later).

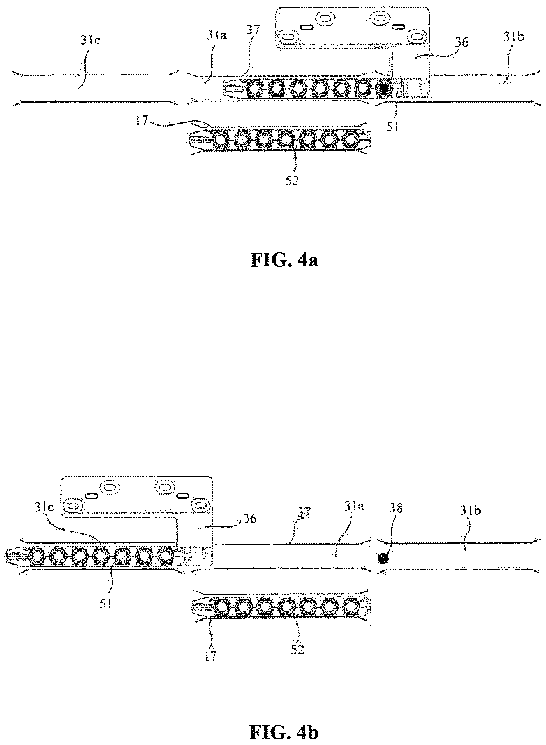

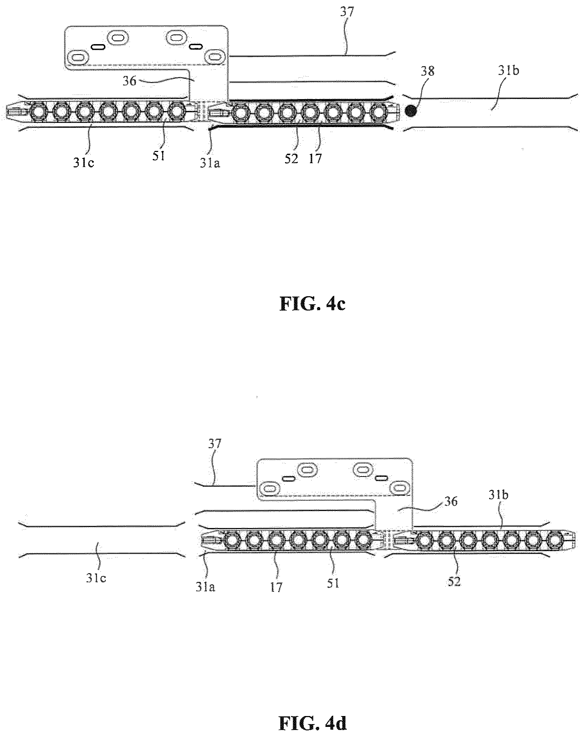

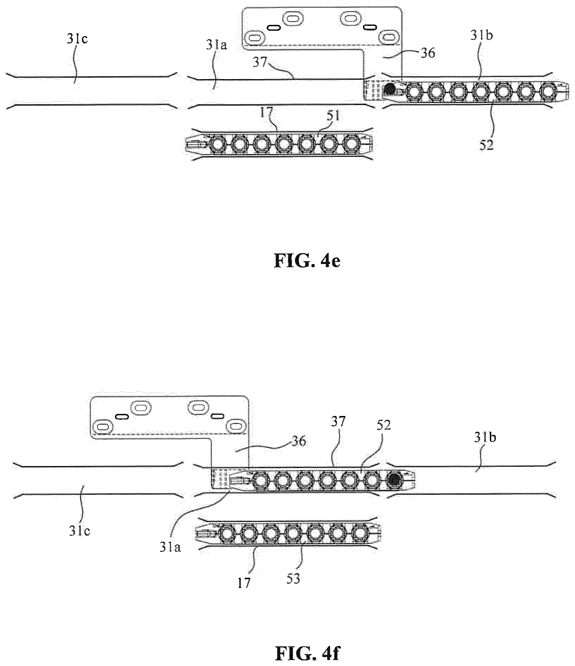

Below, the aspiration channel 31 will be described with reference to FIG. 3. To clearly show the various regions of the aspiration channel 31, the sample rack 50 is not shown in FIG. 3. As shown in FIG. 3, the aspiration channel 31 includes a loading/unloading area 31a, an aspiration area 31b, and a buffer area 31c. The aspiration area 31b and the buffer area 31c are on opposite sides of the loading/unloading area 31a. In other words, the loading/unloading area 31a connects the aspiration area 31b and the buffer area 31c. When loading or unloading the sample rack 50 into/from the aspiration channel 31, the shuttle 17 serves as a passage connecting the aspiration area 31b and the buffer area 31c in the loading/unloading area 31a (as shown in FIGS. 4c and 4d). At times other than loading and unloading the sample rack 50, the channel bridging device 37 serves as a passage connecting the aspiration area 31b and the buffer area 31c in the loading/unloading area 31a (as shown in FIGS. 4a, 4b, 4e, and 4f).

The sample rack manipulation device 36 can operate on two sample racks on the aspiration channel 31 simultaneously, enabling efficient movement of the sample racks between various regions of the aspiration channel 31. Below, the process of the sample analyzer 1 operating on two sample racks during sampling by the sample rack manipulation device 36 will be described with reference to FIGS. 4a to 4f.

Referring to FIG. 4a, the channel bridging device 37 is located in the loading/unloading area 31a and serves as a passage connecting the aspiration area 31b and the buffer area 31c. The sample rack manipulation device 36 is engaged with a first sample rack 51, and sampling is being carried out on the sample in the rightmost sample container on the first sample rack 51 at a sampling point 38 in the aspiration area 31b. Typically, during sampling, the sample containers on the sample rack (e.g., the first sample rack 51) are sampled sequentially. That is, each of the sample containers passes through the sampling point 38 in the aspiration area 31b in sequence via the sample rack manipulation device 36. For example, in the example shown in FIG. 4a, samples in the sample containers on the first sample rack 51 are collected from left to right. When the sample in the rightmost sample container on the first sample rack 51 is being collected, it indicates that the sampling process for the first sample rack 51 is almost completed and that sampling for the next sample rack (e.g., a second sample rack 52) will commence. At this time, the shuttle 17 is carrying the second sample rack 52 and moving towards the aspiration channel 31.

When the shuttle 17 carrying the second sample rack 52 is about to reach the aspiration channel 31, the sample rack manipulation device 36 transports the first sample rack 51, for which sampling has been completed, from the aspiration area 31b to the buffer area 31c for unloading the first sample rack 51, as shown in FIG. 4b.

When the first sample rack 51 reaches the buffer area 31c, freeing up space in the loading/unloading area 31a, the shuttle 17 carrying the second sample rack 52 pushes the channel bridging device 37 away from the aspiration channel 31 and enters the loading/unloading area 31a, as shown in FIG. 4c. At this time, the shuttle 17 serves as a passage connecting the aspiration area 31b and the buffer area 31c in the loading/unloading area 31a. Simultaneously, as the shuttle 17 enters the loading/unloading area 31a, the sample rack manipulation device 36 is engaged with the second sample rack 52. As shown in FIG. 4c, the sample rack manipulation device 36 is engaged with both the first sample rack 51 and the second sample rack 52.

Next, as shown in FIG. 4d, the sample rack manipulation device 36 moves both the first sample rack 51 and the second sample rack 52 towards the aspiration area 31b, positioning the first sample rack 51 in the loading/unloading area 31a for loading onto the shuttle 17 and the second sample rack 52 in the aspiration area 31b for sampling.

After the first sample rack 51 is loaded onto the shuttle 17, the shuttle 17 leaves the aspiration channel 31, while the sample rack manipulation device 36 is disengaged from the first sample rack 51. After the shuttle 17 leaves the aspiration channel 31, the channel bridging device 37 returns to the loading/unloading area 31a of the aspiration channel 31, as shown in FIG. 4e.

The sample rack manipulation device 36 operates to move the second sample rack 52 so that the sample containers on the second sample rack 52 reach the sampling point 38 one by one, allowing the sampling probe 35 of the sampling device 33 to collect samples from the corresponding sample containers. Additionally, the shuttle 17 can perform other tasks in the sample loading/unloading unit 10 of the sample analyzer 1.

After sampling of all sample containers on the second sample rack 52 is completed, as shown in FIG. 4f, the shuttle 17 is loaded with a third sample rack 53 and moves towards the aspiration channel 31. The process shown in FIGS. 4b to 4e is repeated.

It should be understood that the various steps in the above-described operation process on the sample rack can be adjusted as needed and are not limited to the specific examples described herein.

From the above description, it can be seen that the sample rack manipulation device 36 can be engaged with two sample racks simultaneously and operate on both sample racks at the same time. In the example described above with reference to FIGS. 4a to 4f, the sample rack manipulation device 36 is used to load one sample rack and unload another sample rack simultaneously during sample collection. It should be understood that the sample rack manipulation device 36 according to the present application can be used in any suitable situation and is not limited to the specific examples described herein. Since the sample rack manipulation device according to the present application can operate on two sample racks simultaneously, it can significantly improve the operational efficiency on the sample racks and thus enhance the throughput and efficiency of the sample analyzer.

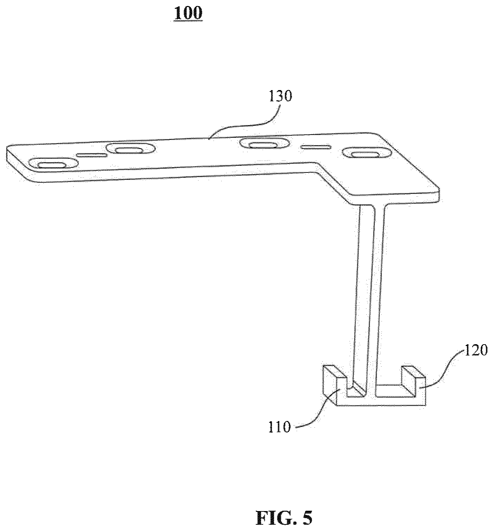

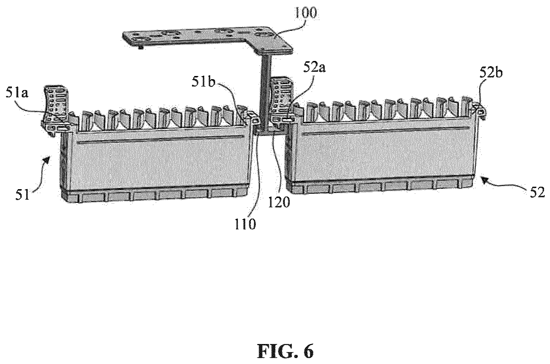

Below, the sample rack manipulation device 100 according to the first embodiment of the present disclosure will be described with reference to FIGS. 5 and 6. FIG. 6 is a perspective schematic view of a sample rack manipulation device 100 according to a first embodiment of the present disclosure, and FIG. 6 is a schematic view illustrating an engagement of the sample rack manipulation device 100 in FIG. 5 with two sample racks 51 and 52.

The sample rack manipulation device 100 shown in FIGS. 5 and 6 is suitable for sample racks available from Beckman Coulter, Inc. It should be understood that the sample racks available from Beckman Coulter are provided as examples for illustrative purposes only. It should be understood that the sample rack manipulation device according to the present application is not limited to the structure of the sample racks shown in the figures but can be adapted to various sample racks or can vary based on the structures of various sample racks, as long as it can be engaged with two sample racks simultaneously and operate on both sample racks at the same time.

As shown in FIG. 6, the first sample rack 51 and the second sample rack 52 are of a same structure. Both the first sample rack 51 and the second sample rack 52 have an elongated shape and are configured with a row of compartments such as cylindrical recesses for holding test-tube-shaped sample containers. The first sample rack 51 has a proximal end 51a which is close to the operator during operation and a distal end 51b. The proximal end 51a and the distal end 51b extend outward from upper ends of opposite side walls and include hook-shaped portions with recesses. Similarly, the second sample rack 52 has a proximal end 52a and a distal end 52b, which have the same structure as the proximal end 51a and the distal end 51b, respectively.

Referring to FIG. 5, the sample rack manipulation device 100 includes a first engagement portion 110, a second engagement portion 120, and a driven portion 130. The first engagement portion 110 and the second engagement portion 120 are configured to be engaged with sample racks. The first engagement portion 110 and the second engagement portion 120 can be engaged with different parts of the sample racks and may have the same or different structures or sizes, depending on the structures of the parts of the sample racks to be engaged.

In FIG. 6, the first engagement portion 110 is engaged with the distal end 51b of the first sample rack 51, and the second engagement portion 120 is engaged with the proximal end 52a of the second sample rack 52. The first engagement portion 110 is configured to be engaged with a recess of the hook-shaped portion of the distal end 51b. The second engagement portion 120 is configured to be engaged with a recess of the hook-shaped portion of the proximal end 52a. Both the first engagement portion 110 and the second engagement portion 120 can be in the form of hook-shaped members.

The first engagement portion 110 and the second engagement portion 120 are arranged in a back-to-back configuration. In this way, the sample rack manipulation device 100 will be more compact. As shown in FIG. 6, when the sample rack manipulation device 100 is engaged with the first sample rack 51 and the second sample rack 52, it occupies relatively little space between the first sample rack 51 and the second sample rack 52.

It should be understood that the structure and arrangement of the first engagement portion 110 and the second engagement portion 120 can vary based on the structures of the sample racks and are not limited to the hook-shaped form shown in the figures. For example, either the first engagement portion or the second engagement portion can be in the form of a clamping member. For example, the first engagement portion and the second engagement portion can be at different horizontal levels or can be spaced apart by a certain distance. For example, the first engagement portion and the second engagement portion can be engaged with the same part of different sample racks.

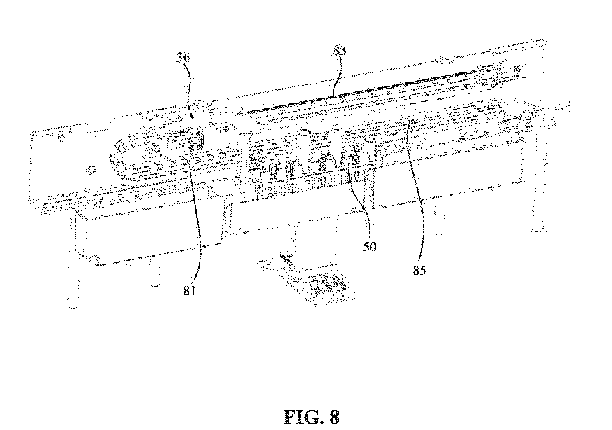

The driven portion 130 can be engaged with a driving device (not shown) and driven by the driving device. For example, the driving device may be a stepping motor (not shown). For example, referring to FIG. 8, the stepping motor is coupled to and drives the driven portion 130 of the sample rack manipulation device 100 via a timing belt 85. The sample analyzer 1 may also include a guide member (such as a guide slot 83 shown in FIG. 8) for guiding the movement of the sample rack manipulation device.

It should be understood that the structure of the driven portion 130 may vary accordingly based on the type and structure of the driving device, as long as the functions described herein can be achieved. Advantageously, the driving device can move the driven portion 130 (i.e., the sample rack manipulation device 100) in two opposite directions.

The sample rack manipulation device 100 can also include sensors (indicated by reference numeral 81 in FIG. 8) for sensing positions. For example, the sample racks can be accurately transported to predetermined positions by sensing positions with the sensors. It should be understood that the type, quantity, and position of the sensors should not be limited but can be changed as needed.

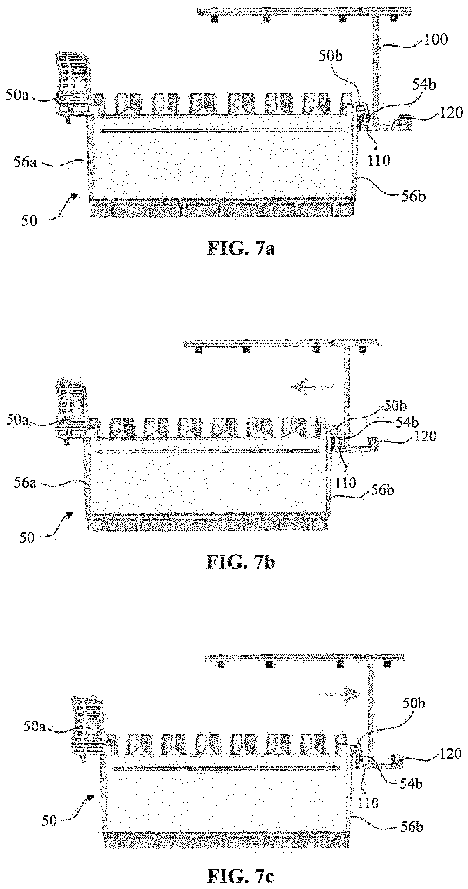

In addition, the sample rack can be accurately positioned by operating the sample rack manipulation device 100. For example, by positioning the sample rack, the corresponding sample container on the sample rack can be accurately placed at the sampling point for successful sampling. Below, the positioning of the sample rack 50 by the sample rack manipulation device 100 will be described with reference to FIGS. 7a to 7c.

Referring to FIG. 7a, the sample rack 50 has the same structure as the sample racks 51 and 52 shown in FIG. 6. Specifically, the sample rack 50 has a front side wall 56a, a rear side wall 56b, a proximal end 50a extending from an upper end of the front side wall 56a, and a distal end 50b extending from an upper end of the rear side wall 56b. A free end of the first engagement portion 110 of the sample rack manipulation device 100 is engaged in the recess of the distal end 50b of the sample rack 50. Generally, a width of the recess in the distal end 50b is greater than a wall thickness of the free end of the first engagement portion 110, so that the first engagement portion 110 can easily enter the recess in the distal end 50b. At this time, there may be a gap between the free end of the first engagement portion 110 and the rear side wall 56b of the sample rack 50 and/or a free cantilever 54b of the distal end 50b. When the first engagement portion 110 is engaged with each of the sample racks 50, the value of this gap may vary, that is, the position of the sample rack 50 may not be accurate at this time.

Referring to FIG. 7b, the sample rack manipulation device 100 is first moved a predetermined distance (also referred to as a first predetermined distance) to the left. This first predetermined distance is greater than the gap between the first engagement portion 110 and the sample rack 50 (specifically, the rear side wall 56b). In this way, after the sample rack manipulation device 100 is moved the first predetermined distance to the left, the sample rack manipulation device 100 abuts against the sample rack 50 (specifically, the rear side wall 56b) and push the sample rack 50 to the left. The first predetermined distance is set to ensure that the sample rack manipulation device 100 can abut against the sample rack 50 (specifically, the rear side wall 56b).

Referring to FIG. 7c, the sample rack manipulation device 100 is then moved a predetermined distance (also referred to as a second predetermined distance) to the right. Similarly, this second predetermined distance is greater than the gap between the first engagement portion 110 and the sample rack 50 (specifically, the free cantilever 54b of the distal end 50b). In this way, after the sample rack manipulation device 100 is moved the second predetermined distance to the right, the sample rack manipulation device 100 abuts against the sample rack 50 (specifically, the free cantilever 54b of the distal end 50b) and push the sample rack 50 to the right. The second predetermined distance is set to ensure that the sample rack manipulation device 100 can abut against the sample rack 50 (specifically, the free cantilever 54b of the distal end 50b). The second predetermined distance may be greater than or equal to the first predetermined distance.

In the example shown in FIGS. 7a to 7c, the positioning of the sample rack 50 is achieved by the abutment between the first engagement portion 110 of the sample rack manipulation device 100 and the sample rack 50. It should be understood that the positioning of the sample rack 50 may also be achieved by the abutment between the second engagement portion 120 of the sample rack manipulation device 100 and the proximal end 50a of the sample rack 50 through the above method. In the above positioning method, only the movement of the sample rack manipulation device 100 in two opposite directions needs to be controlled, and there is no need to additionally set up a device for positioning the sample rack 50, thus reducing the cost of additional positioning devices.

It should be understood that the method for positioning a sample rack by the sample rack manipulation device is not limited to the specific example described with reference to FIGS. 7a to 7c, but may vary as long as the functions described herein can be achieved.

Although various embodiments and variations of the present disclosure have been described above in detail, those skilled in the art should understand that the present disclosure is not limited to the specific embodiments and variations described above, but may include various other possible conjunctions and combinations. Other modifications and variations may be implemented by those skilled in the art without departing from the essence and scope of the present disclosure. All these variations and variants shall fall within the scope of the present disclosure. Moreover, all components described here can be replaced by other technically equivalent components.

## Claims

1. A sample rack manipulation device for a sample analyzer, comprising:
a first engagement portion which can be engaged with a first sample rack;
a second engagement portion which can be engaged with a second sample rack; and
a driven portion which can be engaged with a driving device and driven by the driving device.

2. The sample rack manipulation device according to claim 1, wherein the first engagement portion is adapted to be engaged with a distal end of the first sample rack, and the second engagement portion is adapted to be engaged with a proximal end of the second sample rack.

3. The sample rack manipulation device according to claim 2, wherein the first engagement portion and the second engagement portion are arranged in a back-to-back configuration.

4. The sample rack manipulation device according to any one of claims 1 to 3, wherein the first engagement portion and the second engagement portion are hook-shaped members that are engaged with the first sample rack and the second sample rack respectively.

5. The sample rack manipulation device according to any one of claims 1 to 3, further comprising a sensor for sensing positions.

6. A sample analyzer, comprising the sample rack manipulation device according to any one of claims 1 to 5.

7. The sample analyzer according to claim 6, further comprising a driving device for driving the sample rack manipulation device, wherein the driving device comprises a stepping motor that drives the sample rack manipulation device via a timing belt.

8. The sample analyzer according to claim 6 or 7, wherein the sample rack manipulation device is configured to operate sample racks on an aspiration channel of the sample analyzer, and the aspiration channel is linear and comprises an aspiration area, a loading/unloading area, and a buffer area sequentially.

9. A method for sample collection in a sample analyzer through the sample rack manipulation device according to claim 1, comprising following steps:
moving a first sample rack to an aspiration area of an aspiration channel of the sample analyzer by the sample rack manipulation device for collecting samples, wherein a first engagement portion of the sample rack manipulation device is engaged with the first sample rack;
after the sample collection from the first sample rack is completed, moving the first sample rack to a buffer area of the aspiration channel;
loading a second sample rack into a loading/unloading area of the aspiration channel and engaging a second engagement portion of the sample rack manipulation device with the second sample rack;
moving the first sample rack and the second sample rack by the sample rack manipulation device such that the first sample rack reaches the loading/unloading area;
unloading the first sample rack from the loading/unloading area; and
moving the second sample rack to the aspiration area by the sample rack manipulation device to collect samples.

10. The method according to claim 9, further comprising:
positioning the first sample rack or the second sample rack by the sample rack manipulation device.

11. The method according to claim 10, wherein the positioning is achieved by abutment between the first engagement portion or the second engagement portion of the sample rack manipulation device and the first sample rack or the second sample rack.

12. The method according to claim 11, wherein the step of the positioning comprises the followings:
moving the first sample rack or the second sample rack a first predetermined distance in a first direction by the sample rack manipulation device; and
moving the first sample rack or the second sample rack a second predetermined distance in a second direction opposite to the first direction by the sample rack manipulation device,
wherein the first predetermined distance and the second predetermined distance are greater than a gap between the first engagement portion or the second engagement portion of the sample rack manipulation device and the first sample rack or the second sample rack.

13. The method according to claim 12, wherein the first predetermined distance is equal to the second predetermined distance.

14. The method according to any one of claims 9 to 13, wherein the first engagement portion and the second engagement portion are engaged with a distal end of the first sample rack and a proximal end of the second sample rack respectively, which are adjacent to each other.
